# EUROPEAN PATENT SPECIFICATION

(11) **EP 0 530 338 B1**
(45) Date of publication and mention of the grant of the patent: **02.05.2002**
(21) Application number: 92905799.0
(22) Date of filing: 09.03.1992
(51) Int. Cl.: C07D 333/14, C09K 19/34, C07D 333/26, C09K 19/12, C07C 331/28, C07C 255/55, C07C 255/50, C09K 19/20, C09K 19/18

(54) **ANISOTROPIC ORGANIC COMPOUNDS**
ANISOTROPE ORGANISCHE VERBINDUNGEN
COMPOSES ORGANIQUE ANISOTHROPES

(30) Priority: 13.03.1991 GB 9105362
(43) Date of publication of application: 10.03.1993
(73) Proprietor: QinetiQ Limited, London, SW1 6TD (GB)
(72) Inventor: TOYNE, Kenneth, Johnson, Humberside HU16 7RX (GB); GOODBY, John, William, Humberside HU6 7RX (GB); SEED, Alexander, Humberside HU6 7RX (GB); GRAY, George, William, Wimborne, Dorset BH21 4HD (GB); McDONNEL, Damien, Gerard, Malvern, Worcestershire WR14 3PS (GB); RAYNES, Edward, Peter, Malvern, Worcestershire WR14 3PS (GB); DAY, Sally, Elizabeth, Malvern, Worcestershire WR14 3PS (GB); HARRISON, Kenneth, John, Malvern, Worcestershire WR14 3PS (GB); HIRD, Michael, Humberside HU6 7RX (GB)
(74) Representative: Bowdery, Anthony Oliver
(86) International application number: GB9200412
(87) International publication number: WO9216519

(56) References cited:
- GB-A- 2 229 179
- Chemical Abstracts, volume 111, no. 2, 10 July 1989, (Columbus, Ohio, US) H. Satonaka: "Synthesis and mesomorphic properties of 2-thiophenecarboxylic acid esters and 2-thienylacrylic acid esters", see page 583, abstract 15651x, & Senryo to Yakuhin, 1988, 33(8), 223-38
- Molecular Crystals and Liquid Crystals, volume 123, nos. 1/4, 1985, Gordon and Breach, Science Publishers Inc. and OPA Ltd (US) D.C. McDonnell et al.: "The physical properties of fluorine derivatives of 4-cyanobiphenyls", pages 169-177, see the whole article

## Description

This invention relates to compounds containing a cyano, isothiocyanato or thiocyanate group and which have liquid crystalline properties and/or are suitable for use as constituents of liquid crystal materials. The invention also relates to use of such compounds in liquid crystal materials.

Liquid crystal materials and devices exploit the electro-optical propertiesof nematic and cholesteric (N or N*), or smectic (S) with particularly useful smectic phases being chiral smectic C (S_{c}*) or smectic A.

Liquid crystal materials which show ferroelectric S_{c}* phase are useful in fast switching displays such as television or VDU screens as the S_{c}* phase can be swithched in a few milliseconds or even microseconds. The principle of S_{c}* switching is described *inter alia* by N A Clark and S T Lagerwall in App Phys Lett 36 (1980) p899.

Materials which show an S_{A} liquid crystal phase may be used in display devices which exploit the electroclinic effect.

The use of liquid crystal materials to exhibit electro-optical effects in displays and other optical devices such as digital calculators, watches, meters and alphanumeric laptop computers is now well known. However, known liquid crystal materials are not ideal in all respects and a considerable amount of work is currently being carried out in the art to improve their properties.

Liquid crystal materials normally consist of specially selected mixture compositions and improved materials are often obtained by forming new mixtures having an improved combination of properties.

The composition of a liquid crystal mixture is generally selected so that the mixture shows desirable properties. In particular such properties include:
(1) a liquid crystalline temperature range - including room temperature (20°C) - which is as wide as possible;
(2) a melting point (solid-to-liquid crystal transition temperature) which is as low as possible;
(3) a clearing point (liquid crystalline to isotropic liquid transition temperature) which is as high as possible;
(4) a positive or negative (as appropriate) dielectric anisotropy (permittivity measured parallel to the molecular axis less that measured perpendicular to the molecular axis) which is as great as possible in order to minimise the display voltage;
(5) a viscosity which is as low as possible in order to minimise the display switching speeds;
(6) an electro-optical response which varies as little as possible with temperatures;
(7) a good chemical and photochemical stability;

Examples of further particular properties useful in specific applications include:
(8) a good multiplexability;
(9) an ability to switch dielectric anisotropy with frequency;
(10) a birefringence of selected magnitude;
(11) specific elastic constants which can be tailored to meet specific device requirements; and
(12) high electrical resistivity for certain applications.

Liquid crystal materials are generally mixtures of compounds which individually or together show a liquid crystal phase. A number of desirable characteristics are sought in such compounds and materials. Among these are chemical stability, persistence of appropriate liquid crystal phases over a wide temperature range preferably including room temperature, and for some devices a high birefringence (Δn) is sought.

A class of compounds which is widely used as nematic materials are the alkyl and alkoxy cyanobiphenyls and terphenyls: 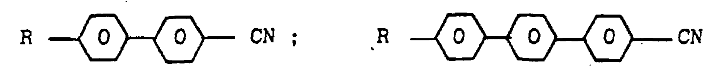 where R is alkyl or alkoxy. These are useful liquid crystalline compounds, but for some applications compounds with higher birefringence is dersirable.

Two items of prior art that are relevant to this invention are GB-A-2229179 and C.A. 111: 15651x.

It is an object of this invention to provide compounds and materials having at least some of these desirable characteristics, and in particular a high birefringence.

According to a first aspect this invention provides liquid crystalline compounds of formula I: 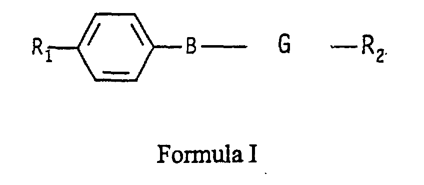 wherein R₁ is selected from alkyl, alkoxy, alkynyl, thioalkyl, hydrogen, NCS and SCN, R₂ is selected from alkyl, alkoxy, alkynyl, thioalkyl, hydrogen, CN, NCS and SCN; wherein alkyl, alkoxy, alkynyl, thioalkyl groups have from 1 to 15 carbon atoms; G is thiophene; B is selected from single bond, C≡C, C=C, COO, azoxy and diazo; provided that at least one of R₁ and R₂ is selected from SCN or NCS.

The structural and other preferences are expressed below on the basis of *inter alia* desirable liquid crystalline characteristics, in particular high birefringence for use in liquid crystal materials.

According to a second aspect this invention provides liquid crystalline compounds of formula I wherein R₁ is CN, R₂ is selected from NCS and SCN; G is thiophene, B is selected from single bond, C≡C, C=C, azoxy and diazo.

Typically such characterisitics can be utilised in devices such as Optical Kerr Effect devices. Such devices are often used as optical shutters or optical modulators, and rely on the fact that birefringence (Δn) of a medium is proportional to the square of an applied electric field. Such an effect is often termed the quadratic electro-optic effect and can be investigated using degenerate four wave mixing (P Madden et al IEEE J of Quantum Electronics QE22 No 8 Aug 1986 p1287).

Compounds of formula I can be prepared by various routes which will be apparent to those in the art. A preferred route for compounds of formula I where R₁ is NCS comprises coupling of the appropriate boronic acid with 1-Bromo-nitrobenzene mediated by a palladium catalyst (eg tetrakis (triphenylphosphine)palladium(O)) to achieve the appropriate nitrophenyl. Conversion of the nitrophenyl to isothiocyanatophenyl is possible via hydrogenation to a phenyl amine with subsequent treatment with thiophosgene. Suitable conditions for such couplings are well known.

Typical preparation routes for compounds of formula I where R₁ is CN include coupling of the appropriate boronic acid with 4-Bromonitrobenzile, typically mediated by a palladium catalyst. Inclusion of linking groups B are well known to those skilled in the art as are the methods of the preparation of the appropriate boronic acids to include the required end group substituent.

According to a third aspect this invention provides a liquid crystal material, the material being a mixture of compounds at least one of which is a compound according to formula I.

Compounds of formula I have a number of desirable properties which make them very useful components of liquid crystal materials, particularly their high birefringence.

Suitable compounds for the other components of the liquid crystal materials will be apparent to those skilled in the field, and will depend upon the properties such as dielectric anisotropy, birefringence, working temperature range etc required in the material for the application for which the material is intended. Some types of suitable material are discussed briefly below.

Preferably, as well as containing at least one of the formula I compounds, the liquid crystalline material of the invention contains one or more compounds: 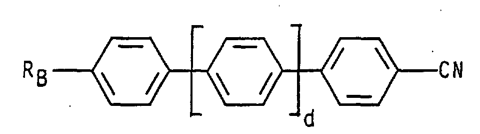 wherein R_{B} is alkyl, thioalkyl or alkoxy, preferably containing 1-8 carbon atoms, and preferably straight chain, and wherein d is 1 or 0.

Such materials are included in the subject matter of GB 1433130 and GB 2238306. The liquid crystal material may for example contain other liquid crystalline compounds which have a positive dielectric anisotropy, for example as described in EP-A-01322377.

The liquid crystal material of the invention may also contain compounds having the formula: 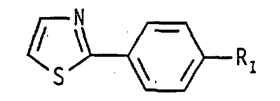 wherein R₁ is CN, alkyl, alkoxy.

The material may alternatively or also contain liquid crystalline compounds of low dielectric anisotropy, or a cholesteric mixture which may be thermochromic. Some examples of such compounds are described in EP-A-0132377.

The material may alternatively or also contain liquid crystalline compounds having a high clearing point, for example in order to raise the nematic phase to isotropic phase (N-I) transition temperature. Some examples of such compounds are described in EP-A-0132377.

To cause the material of this aspect of the invention to show a cholesteric or chiral nematic phase the material must contain at least one compound containing an asymmetric carbon atom. This may be a chiral compound of formula I, eg S(+) 4-(2-methylbutyl)-4'-cyano biphenyl or S( +) 4- (2-methylbutoxy) - 4'-cyano biphenyl.

The material may also contain one or more pleochroic dyes, for example the dyes described in EP-A-59095.

The proportions of these components used in the material of this aspect of the invention will depend upon the intended application, and the material may usefully contain two or more compounds of formula I. If the material does contain two or more compounds of formula I then they may be in proportions that are approximate to a eutectic mixture.

The materials of this aspect of the invention may be used in many of the known forms of liquid crystal display devices, for example a twisted nematic device, Freedericks effect device, cholesteric memory mode device, cholesteric to nematic phase change effect device, dynamic scattering effect device, or a supertwist effect device. The method of construction and operation of such devices, and characteristics of a liquid crystal material suitable for use therein, are well known in the field.

According to a fourth aspect the invention provides a liquid crystal device which uses a liquid crystal material according to the invention.

According to a fifth aspect the invention provides a liquid crystal device utilising pretransitional characteristics comprising a liquid crystal compound according to formula I.

According to a sixth aspect the invention provides a liquid crystal device utilising pretransitional characteristics comprising a liquid crystal material according to the invention.

Typically an electro-optical display device will consist of 2 substrates between which a layer of the liquid crystal material may be sandwiched. At least one of the substrates is optically transparent and both have addressable electrodes which are preferably made of a transparent material on their opposing faces. By applying an electric field across the layer of liquid crystal material via the electrodes an electro-optical effect is achieved which may be viewed directly or preferably through one or more polarising filters.

Compounds of formula I and also materials including compound(s) of formula II may be used in devices that utilise the optical Kerr Effect. Typically optical Kerr effect devices comprise a glass cell containing two electrodes, where the glass cell is filled with a polar liquid. The device is frequently termed a Kerr cell. The Kerr cell can be positioned between two crossed polarisers having transmisssion axes at ±45° to an electric field applied across the Kerr cell. With zero voltage applied across the Kerr cell no light will be transmitted and the cell operates as a closed shutter. Application of a modulating voltage generates a field causing the Kerr cell to function as a variable wave plate and thus operating the Kerr cell as a shutter capable of opening proportionately to the applied field. Nematic materials of this invention may be particularly suitable for use in ECB effect devices, due to the high birefringence of the materials. They may also be particularly suitable for use in polymer dispersed liquid crystal (PDLC) materials in which small droplets of a liquid crystal material are dispersed within a matrix of a transparent polymer.

Non-limiting examples illustrating this invention will now be given, with reference to figures 1-9 giving typical synthesis routes for example compounds of formula I. Examples 1, 2, and 4 to 8 do not fall within the scope of this invention. Data for 26 compounds are presented in Table 1. Numbering these compounds from E1 (at the top of the table) to E26 (at the bottom of the table); compounds E1, E2, E4 to E10, E12 to E16, E19, E23, and E25 do not fall within the scope of this invention. Finally, Fig. 1, Fig. 2, and Fig. 4 to Fig. 8 also do not fall within the scope of this invention. Figures 10 and 11 are given by way of example only and schematically represent a liquid crystal device of the invention and a Kerr cell of the invention respectively.

### Example 1. Preparation of:

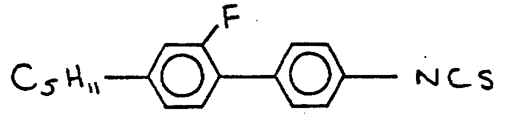

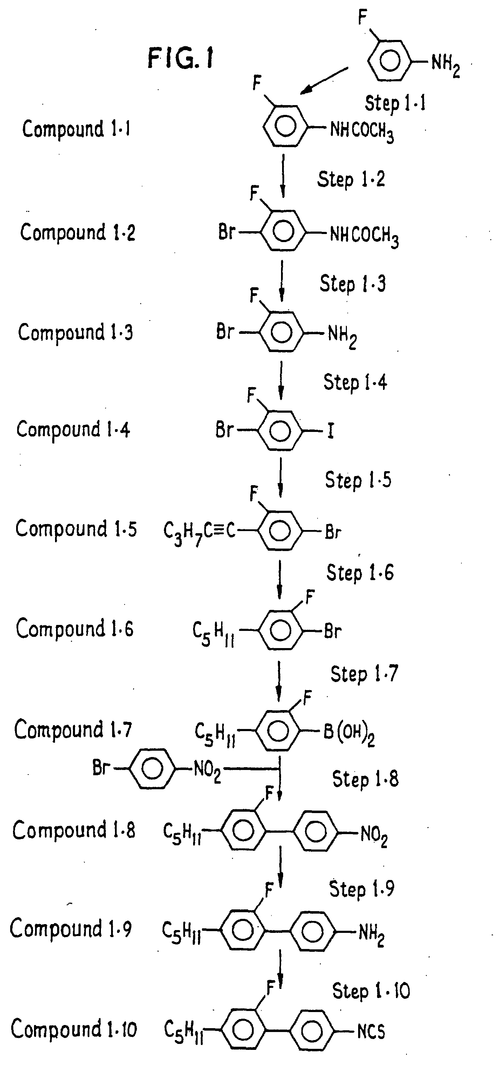

With reference to figure 1 it can be seen that example 1 can be prepared using the following synthetic route.

### Step 1.1 3-Fluoroacetanilide.

A solution of acetic anhydride (30.30g, 0.297mol) in glacial acetic acid (30ml) is added to stirred 3-fluoroaniline (30.08, 0.27mol). The resulting stirred mixture is heated under reflux for 20 minutes and poured into cold water (500ml). the product is extracted into ether (x2) and the combined ethereal extracts are washed with water and dried (MgSO₄). The solvent is removed *in vacuo* and the residue is recrystallized from aqueous acetic acid to yield colourless crystals with a yield of 28.90g (70%).

### Step 1.2 4-Bromo-3-fluoroacetanilide

*N*-Bromosuccinimide (15.73g, 0.088mol) is added all at once to a stirred solution of compound 1.1 (13.50g, 0.088mol) in dry dichloromethane at room temperature. The stirred mixture is heated under reflux for 5 hours (glc analysis showing a complete reaction and the presence of only one product peak) cooled and washed with lots of water. The aqueous extract is washed with dichloromethane and the combined organic extracts are washed with water and dried (MgSO₄). The solvent is removed *in vacuo* to give a pale orange solid, and a further sample can then recrystallised from hexane/dimethoxyethane (99:1) to yield colourless crystals, giving a yield of 20.40g (100%).

### Step 1.3 4-Bromo-3-fluoroaniline

36% hydrochloric acid (25ml) is added dropwise to a stirred, refluxing solution of compound 1.2 (19.75g, 0.085mol) in ethanol (50ml). The solution is heated under reflux for 2 hours (glc analysis revealing a complete reaction), cooled and water then added. The mixture is distilled to remove ethanol and ethylacetate, with the residue added to 5% sodium hydroxide and the product extracted into dichloromethane (x2). The combined organic extracts are washed with water and dried (MgSO₄). The solvent is removed *in vacuo* to give a fawn solid with a yield of 15.95g (99%).

### Step 1.4 1-Bromo-2-fluoro-4-iodobenzene

A stirred mixture of compound 1.3 (13.68g, 0.072mol) and 36% hydrochloric acid (110ml) is gently warmed to obtain a solution, then cooled to -5°C and a solution of sodium nitrite (5.47g, 0.079mol) in water is added dropwise whilst maintaining the temperature at -5°C. The mixture is stirred at 0°C for 30 minutes, with cyclohexane (100ml) then added followed by addition of a solution of potassium iodide (43.5g, 0.26mol) in water dropwise at a temperature of between 0 and 5°C. The mixture is stirred at room temperature for a few hours and the product then extracted into ether (x2). The combined organic extracts are washed with sodium metabisulphite, 10% sodium hydroxide, water and dried (MgSO₄). The solvent is removed *in vacuo* to give an off-white solid with a yield of 35.0g (87%).

### Step 1.5 1-Bromo-2-fluoro-4-pent-1-ynylbenzene

Quantities: pent-1-yne (4.02g, 0.059mol), n-butyllithium (6.00ml, 10.0M in hexane, 0.060mol), zinc chloride (8.16g, 0.060mol), compound 1.4 (15.5g, 0.051mol), tetrakis (triphenylphosphine)palladium(O) (2.95g, 2.55mol).
This experimental procedure is a zinc coupling reaction. The n-butyllithium solution is added dropwise to a stirred, cooled (-5°C to 0°C) solution of the pent-1-yne in dry THF under dry nitrogen. This mixture is stirred for 10 minutes and then a solution of the zinc chloride (dry) in dry THF is added dropwise at about -5°C to 0°C. The mixture is stirred at room temperature for 15 minutes and a solution of compound 1.2 in dry THF is added dropwise at -5°C to 0°C followed by addition of the tetrakis (triphenylphosphine)palladium(0). The mixture is heated under reflux for 22 hours (glc analysis revealing a complete reaction). The crude product is distilled to yield 11.31g (92%) colourless liquid.

### Step 1.6 1-Bromo-2-fluoro-4-pentylbenzene

A stirred mixture of compound 1.5 (10.95g, 0.045mol) and platinum (IV) oxide (0.25g) in ethanol (150ml) is hydrogenated at room temperature and atmospheric pressure for 8 hours (glc analysis showing a complete reaction). The catalyst is filtered off and the solvent is removed *in vacuo* to yield a pale-orange solid with a yield of 10.58g (98%).

### Step 1.7 2-Fluoro-4-pentylphenylboronic acid

Quantities: compound 1.6 (9.92g, 0.040mol), n-butyllithium (4.00ml, 10.0M in hexane, 0.040mol), trimethyl borate (8.35g, 0.080mol).
This experimental procedure is a standard boronic acid preparation and yields a brown solid. The yield is 8.28g (99%).

### Step 1.8 2-Fluoro-4'-nitro-4-pentylbiphenyl

Quantities: 1-Bromo-4-nitrobenzene (2.15g, 0.0106mol), compound 1.7 (2.68g, 0.0128mol), tetrakis(triphenylphosphine)palladium(0) (0.38g, 0.33mol).
This experimental procedure nitration where the crude product is purified by column chromatography [silica gel/ petroleum fraction (bp 40-60°C) - dichloromethane, 3:1] to give a pale yellow gel with yield of 2.95g (97%).

### Step 1.9 4'-Amino-2-fluoro-4-pentylbiphenyl

A stirred mixture of compound 1.8 (2.80g, 9.76mol) and 5% palladium on charcoal (1.50g) in ethanol (100ml) is hydrogenated at room temperature and atmospheric pressure for 8 hours (when glc analysis revealed a complete reaction). The catalyst is filtered off and the solvent removed *in vacuo* to yield 15.35g (95%) of pale orange solid.

### Step 1.10 2-Fluoro-4'-isothiocyanato-4-pentylbiphenyl

A solution of compound 1.9 (2.11g,8.21mol) in chloroform is added to a stirred, cooled (0°C) mixture of water, calcium carbonate (1.28g, 0.013mol), chloroform and thiophosgene (1.17g, 0.010mol). The mixture is heated at 35°C for 1.5 hours and then poured into water. The aqueous layer is washed with dichloromethane and the combined organic extracts are washed with 1% aqueous hydrochloric acid and dried (MgSO₄). The solvent is removed *in vacuo* and the crude product purified by column chromatography (silica gel/dichloromethane) to give an off-white solid which is then crystallised from ethanol-ethyl acetate (1:1) to yield 1.36g (55%) colourless crystals.

### Example 2. Preparation of:

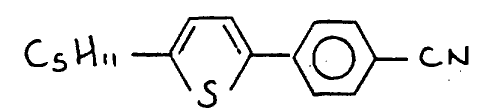

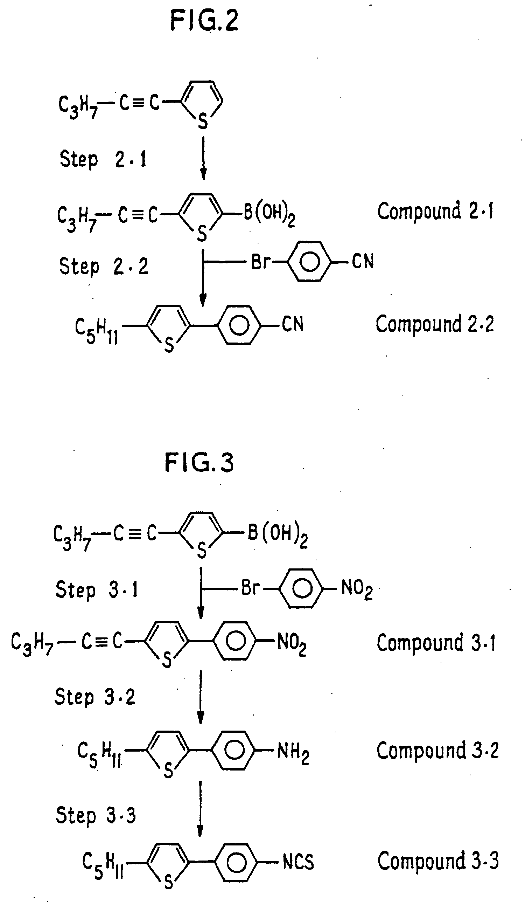

With reference to figure 2 it can be seen that example 2 can be prepared using the following synthetic route.

### Step 2.1 2-Pent-1-ynylthiophene.

A solution of n-butyllithium (10.0ml, 10.0M in hexane, 0.10mol) is added dropwise to a stirred cooled (-5 to 0°C) solution of pent-1-yne (6.80g, 0.10mol) in dry THF under dry nitrogen. This mixture is stirred for 10 minutes and then a solution of dry zinc chloride (13.63g, 0.10mol) in dry THF is added dropwise to 2-bromothiophene (16.00g, 0.098mol) at a temperature of between -5°C and 0°C followed by addition of tetrakis (triphenylphosphine)palladium(0) (3.40g, 2.94mol). The mixture is stirred at room temperature overnight (glc analysis revealing a complete reaction) and poured into 10% hydrochloric acid. The product is extracted into ether (x2) and the combined ethereal extracts are washed with aqueous sodium hydrogen carbonate and dried (MgSO₄). The solvent is removed *in vacuo,* then the product is filtered and distilled to yield 12.96g (88%) colourless liquid.

### Step 2.2 5-pent-1-ynylthiopen-2-ylboronic acid.

This experimental procedure is a standard boronic acid preparation using compound 2.1 (10.00g, 0.067mol), n-butyllithium (6.80ml, 10.0M in hexane, 0.068mol) and trimethyl borate (14.20g, 0.137mol). The yield is 12.00g (93%) of brown solid.

### Step 2.3 2-(4-cyanatophenyl)-5-pentylthiophene.

This is a standard coupling reaction where a compound 2.2 (1.85g, 9.54mol), 4-benzonitrile (1.45g, 7.97mol) and tetrakis (triphenylphosphine) palladium(0) (0.30g, 0.26mol) are used with 1,2-dimethoxymethane (35ml) and 2M-sodium carbonate (35ml) as solvents. The crude product is purified by column chromatography [silica gel/petroleum fraction (bp40-60°C) - dichloromethane, 2:1] and hydrogenated in ethanol (100ml) in the presence of 5% palladium on charcoal catalyst at room temperature and atmospheric pressure. The catalyst is filtered off and the solvent removed *in vacuo* and the residue purified by [silica gel/ petroleum fraction (bp40-60°C) - dichloromethane, 2:1] to give a colourless oil which is subsequently distilled [Kugelrohr 150°C(max) at 0.55mmHg] to yield a colourless oil which crystallises on cooling. Yield is 1.45g (71%).

### Example 3. Preparation of:

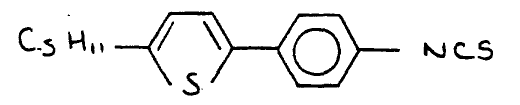

With reference to figure 3 it can be seen that example 3 can be prepared using the following synthetic route.

### Step 3.1 2-(4-nitrophenyl)-5-pent-1-ynylthiophene.

Quantities: 1-Bromo-4-nitrobenzene (2.35g, 0.012mol), compound 2.2 (2.75g, 0.014mol), tetrakis(triphenylphosphine)palladium(O) (0.42g, 0.36mol).
This experimental procedure is a standard nitration where the crude product is purified by column chromatography [silica gel/ petroleum fraction (bp 40-60°C) - dichloromethane, 3:1] to give a pale yellow gel with yield of 2.64g (81%).

### Step 3.2 2-(4-Aminophenyl)-5-pentylthiophene.

This procedure is a standard hydrogenation as exemplified by step 1.9 above, and uses compound 3.1 (2.42g, 8.93mol) and 5% palladium on charcoal (2.00g). A pale yellow solid results with a yield of 2.13g (97%).

### Step 3.3 2-(4-isothiocyanatophenyl)-5-pentylthiophene.

A solution of compound 3.2 (1.98g, 8.08mol) in chloroform is added to a stirred, cooled (0°C) mixture of water, calcium carbonate (1.26g, 0.0126mol), chloroform and thiophosgene (1.16g, 0.010mol). The mixture is heated at 40°C for 2 hours and then poured into water. The product is extracted into dichloromethane and the combined organic extracts are washed with 10% aqueous hydrochloric acid and dried (MgSO₄). The solvent is removed *in vacuo* and the crude product purified by column chromatography (silica gel/dichloromethane) to give an off-white solid which is then crystallised from ethanol to yield 1.63g (70%) colourless crystals.

### Example 4. Preparation of:

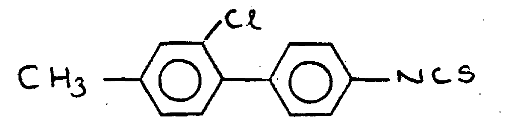

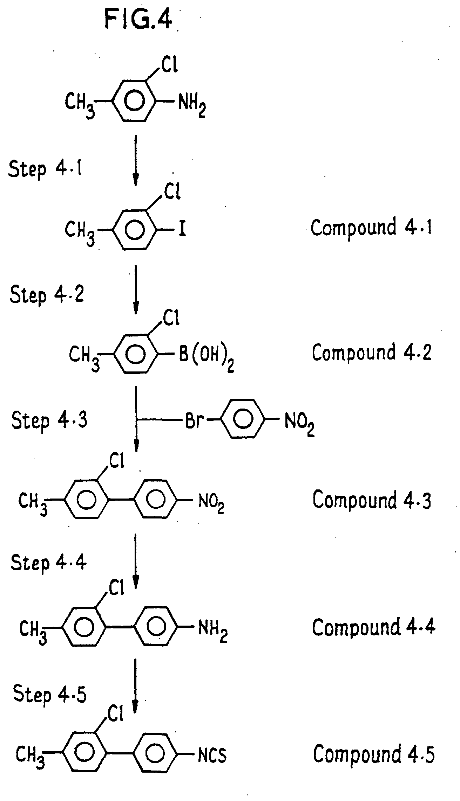

With reference to figure 4 it can be seen that example 4 can be prepared using the following synthetic route.

### Step 4.1 3-chloro-4-iodotoluene.

A stirred mixture of 2-chloromethylaniline (25.00g, 0.177mol) and 36% hydrochloric acid is warmed gently to obtain a solution, then cooled to -5°C and a solution of sodium nitrite (13.45g, 0.195mol) in water is added dropwise whilst maintaining the temperature at -5°C. The mixture is stirred at 0°C for 30 minutes, 100ml of cyclohexane is added and a solution of potassium iodide (58.77g, 0.354mol) in water is added dropwise at a temperature of between 0 and 5°C. The mixture is stirred at room temperature (overnight for convenience) and the product is extracted into ether (x2). The combined organic extracts are washed with sodium metabisulphite, 10% sodium hydroxide, water and dried (MgSO₄). The solvent is *in vacuo* to yield 35.0g (87%) off-white solid.

### Step 4.2 2-Chloro-4-methylphenylboronic acid.

This experimental procedure is a standard boronic acid preparation using compound 4.1 (18.50g, 0.073mol), n-butyllithium (7.50ml, 10.0M in hexane, 0.075mol) and trimethyl borate (15.20g, 0.146mol). The crude product is extracted into 10% potassium hydroxide solution and then washed with ether. The separated aqueous extract is acidified with 36% hydrochloric acid and the product extracted into ether (x2). The combined ethereal extracts are washed with water and dried (MgSO₄). The solvent is dried *in vacuo* to yield 4.86g (39%) of colourless solid.

### Step 4.3 2-Chloro-4-methyl-4'-nitrobiphenyl

Quantities: 1-Bromo-4-nitrobenzene (2.45g, 0.012mol), compound 4.2 (2.25g, 0.013mol), tetrakis(triphenylphosphine)palladium(0) (0.42g, 0.36mol).
This experimental procedure is a standard nitration where the crude product is purified by column chromatography [silica gel/ petroleum fraction (bp 40-60°C) - dichloromethane, 5:1] to give a very pale yellow solid with yield of 2.95g (99%).

### Step 4.4 4'-Amino-2-chloro-4-methylbiphenyl.

This procedure is a standard hydrogenation using compound 4.3 (2.91g, 0.012mol), platinum(IV)oxide (0.15g), ethyl acetate (60ml) and ethanol (60ml) and as exemplified by steps 1.9 and 3.2. The procedure yields 2.60g (100%).

### Step 4.5 2-Chloro-4'-isothiocyanato-4-biphenyl.

This is a standard procedure as described in step 3.3 and using compound 4.4 (2.41g, 0.011mol) with thiophosgene (1.59g, 0.014mol) and calcium carbonate (1.73g, 0.017mol). The crude product is purified by column chromatography (silica gel/dichloromethane) to give an off-white solid which is then crystallised from ethanol to yield 2.10g (74%) colourless crystals.

### Example 5. Preparation of:

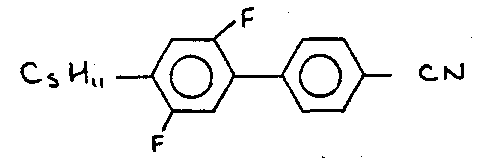

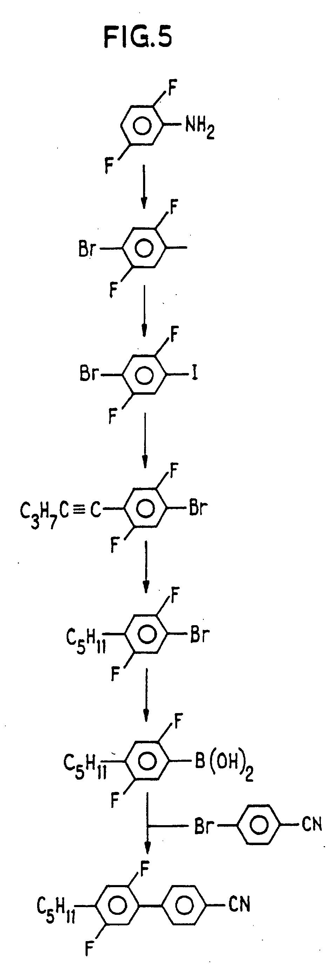

With reference to figure 5 it can be seen that example 5 can be prepared using the following synthetic route.

### Step 5.1 4-Bromo-2.5-difluoroaniline.

*N*-Bromosuccinimide (28.50g,0.160mol) is added in small quantities over 1.5 hours to a stirred, cooled (-10 to 0°C) solution of 2,5-difluoroaniline (20.00g, 0.155mol) in dry dichloromethane under dry nitrogen. This mixture is stirred at 0°C for 2 hours (glc analysis revealing a complete reaction), and the red solution then washed with a large amount of water (x2) and dried (MgSO₄). The solvent is removed *in vacuo* to afford a red solid with a yield of 32.19g (100%).

### Step 5.2 1-Bromo-2.5-difluoro-4-iodobenzene.

Quantities: compound 5.1 (19.00g, 0.091mol), sodium nitrite (7.22g, 0.105mol), potassium iodide (30.25g, 0.182mol)
This procedure is carried out as described for step 1.4 to yield 23.74g (82%) solid.

### Step 5.3 1-Bromo-2.5-difluoro-4-pent-1-ynylbenzene.

Quantities: pent-1-yne (4.90g, 0.059mol), n-butyllithium (7.20ml, 10.0M in hexane, 0.072mol), zinc chloride (9.80g, 0.072mol), compound 5.2 (20.0g, 0.063mol), tetrakis (triphenylphosphine)palladium(0) (1.10g, 0.95mol).
The experimental procedure is as described above for step 1.5 yielding 13.58g (83%).

### Step 5.4 1-Bromo-2.5-difluoro-4-pentylbenzene.

Quantities: compound 5.3 (12.60g, 0.049mol), platinum (IV) oxide (0.20g), ethanol (150ml).
This is an experimental procedure as described above in step 1.6, and yields 12.60g (98%).

### Step 5.5 2.5-Difluoro-4-pentylphenylboronic acid.

Quantities: compound 5.4 (11.90g, 0.045mol), n-butyllithium (4.50ml, 10.0M in hexane, 0.045mol), trimethyl borate (9.40g, 0.090mol).
This experimental procedure is a standard boronic acid preparation and yields a brown solid. The yield is 7.76g (77%).

### Step 5.6 4'-Cyano-2.5-difluoro-4-pentylbiphenyl.

Quantities: 4-Bromonitrobenzile (0.96g, 5.27mol), compound 5.5 (1.38g, 6.05mol), tetrakis(triphenylphosphine)palladium(0) (0.19g, 0.16mol).
This experimental procedure is as described above in step 2.3.

### Example 6. Preparation of:

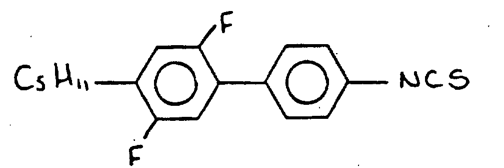

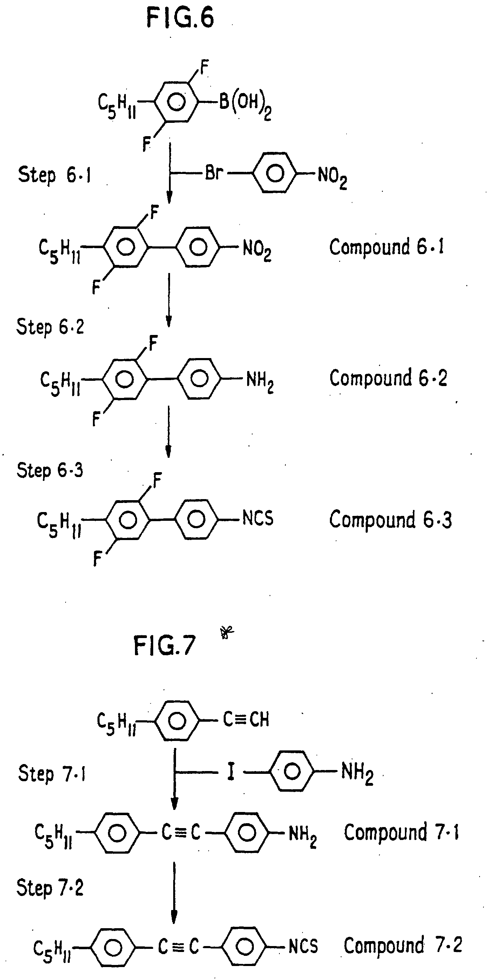

With reference to figure 6 it can be seen that example 6 can be prepared using the following synthetic route.

Preparation of the appropriate boronic acid is carried out in the procedures described above in steps 5.1 to 5.5.

### Step 6.1 2.5-Difluoro-4'-nitro-4-pentylbiphenyl.

Quantities: 1-Bromo-4-nitrobenzene (1.25g, 6.19mol), compound 5.54 (1.55g, 6.80mol), tetrakis(triphenylphosphine)palladium(O) (0.22g, 0.19mol).
This experimental procedure is a standard nitration where the crude product is purified by column chromatography [silica gel/ petroleum fraction (bp 40-60°C) - dichloromethane, 5:1] to give a pale yellow oil with yield of 1.88g (100%).

### Step 6.2 4'-Amino-2.5-difluoro-4-pentylbiphenyl.

Quantities: compound 6.1 (1.75g, 5.74mol), 5% Pd/C (1.30g), ethanol (150ml). This is a standard hydrogenation procedure as exemplified by step 1.9. yield is 1.57g (99%).

### Step 6.3 2.5-Difluoro-4'-isothiocyanato-4-biphenyl.

Quantities: compound 6.2 (1.47g, 5.35mol), thiophosgene (0.78g, 6.78mol), calcium carbonate (0.87g, 8.70mol).
This experimental procedure is as described in step 1.10 giving a yield of 0.80g (47%) colourless crystals.

### Example 7. Preparation of:

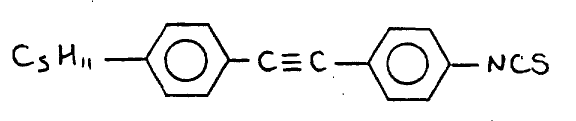

With reference to figure 7 it can be seen that example 7 can be prepared using the following synthetic route.

### Step 7.1 1-(4-Aminophenyl)-2-(4-pentylphenyl)ethyne.

Quantities: 4-pentylphenylethyne (2.18g, 0.0127mol), n-butyllithium (5.10ml, 2.5M in hexane, 0.0127mol), zinc chloride (1.74g, 0.0128mol), 4-iodoaniline (2.5g, 0.011mol), tetrakis(triphenylphosphine)palladium(O) (0.38g, 0.33mol).
This experimental procedure is a zinc chloride coupling reaction as described above in step 1.5.

### Step 7.2 1-(4-isothiocyanatophenyl)-2-(4-pentylphenyl)ethyne.

Quantities: compound 7.1 (2.38g, 9.05mol), thiophosgene (2.38g, 9.08mol), calcium carbonate (1.45g, 0.015mol).
This experimental procedure is as described in step 1.10 giving a yield of 2.23g (81%) colourless crystals.

### Example 8. Preparation of:

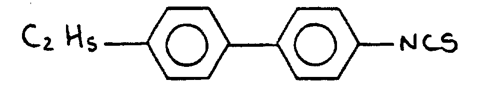

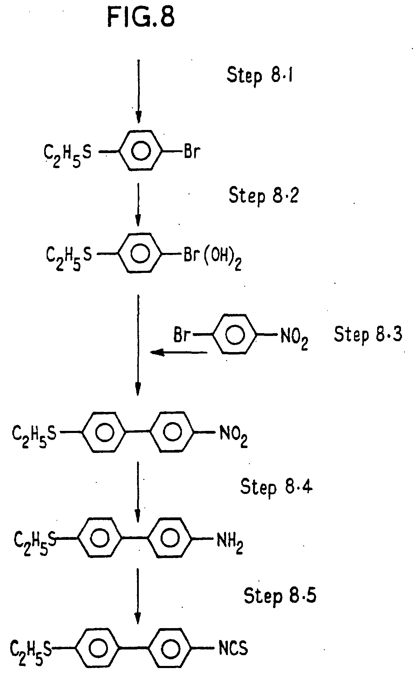

With reference to figure 8 it can be seen that

### Step 8.1 1-Bromo-4-thioethylphenyl.

Bromoethane (70.15g, 0.644mol) was added to a stirred solution of 4-bromobenzenethiol (27.57g, 0.146mol) in sodium ethoxide (3.45g of sodium metal in 100 ml of super-dry ethanol) at room temperature. The solution was heated at 80°C for 2 h (glc analysis confirmed a complete reaction) and the sodium bromide was filtered off. The solvent was removed *in vacuo* and the residue was distilled to give a colourless liquid.
Yield 29.40 g (93%), bp 136-137°C at 20mmHg.

### Step 8.2 4-thioethylphenylboronic acid

Butyllithium (5.1 ml, 10.0M in hexane 0.051 mol) was added dropwise to a stirred, cooled (-78ºC) solution of step 8.1 (10.02 g, 0.046 mol) in dry THF (120 ml) under dry nitrogen at -78°C. The reaction mixture was maintained under these conditions for a further 0.5 h (glc analysis confirmed a complete reaction) before a previously cooled solution of trimethyl borate (10.40 g, 0.10mol) in dry THF was added dropwise at -78°C. The reaction mixture was allowed to warm to room temperature (overnight) and stirred for 1 h with hydrochloric acid (100 ml, 10%) before the product was extracted into either (2 x 200 ml), washed with water and dried (MgSO₄). The solvent was removed *in vacuo* to afford a white solid which was used in the next step without purification.
Yield 17.29 g (95%), mp 88-90°C.

### Step 8.3 4-thioethyl-4'-nitrobiphenyl

1-Bromo-4-nitrobenzene (7.56 g, 00.37 mol) was added all at once to a rapidly stirred mixture of tetrakis (triphenylphosphine) palladium(O) (1.73 g, 0.001 mol), step 8.2 (7.81 g, 0.042 mol), and aqueous sodium carbonate solution (43 ml, 2.0M, 0.09 mol) in dimethoxyethane (50ml), under dry nitrogen. The reaction mixture was refluxed overnight (tlc and glc analysis revealed a complete reaction) and the product was extracted into ether (2 x 200 ml); the combined ethereal solutions were washed with saturated sodium chloride solution (300 ml) and dried (MgSO₄). The solvent was removed *in vacuo* and the product was purified by column chromatography [silica gel/petroleum fraction (bp 40-60°C), dichloromethane, 5:1] and was recrystallised from ethanol to afford a pale yellow solid which was dried *in vacuo* (P₂O₅).
Yield 6.51 g (60%), mp 86.2-86.4°C.

### Step 8.4 4'Amino-4-thioethylbiphenyl

A stirred solution of step 8.3 (5.16 g, 0.020 mol) and palladium on carbon (5%, 1.96 g), in ethanol (30 ml) and tetrahydrofuran (30 ml), was stirred under hydrogen overnight. The catalyst was removed by filtration through 'Hyflo supercel' and the solvent was removed *in vacuo* to afford a purple solic which was used in the next step without purification.
Yield 4.61 g (100%).

### Step 8.5 4-thioethyl-4'-isothiocyanatobiphenyl

A solution of step 8.4 (4.61 g, 0.020 mol) in chloroform (75 ml) was added to a stirred, cooled (0-5°C) solution of calcium carbonate (2.59g, 0.026mol) and thiophosgene (2.30g, 0.020mol) in water (40ml) and chloroform (20ml) at 0-5°C. The mixture was heated at 35°C for lh (glc and tlc analysis confirmed a complete reaction) and poured into water (50ml). The organic layer was washed with hydrochloric acid (1%, 100ml) and dried (MgSO₄). The compound was purified by column chromatography [silica gel/ petroleum (bp40-60°C), dichloromethane, 5:1] and was recrystallised to give 0.57g (22% yield) of a white solid which was dried *in vacuo* (CaCO₃), having an indicated (hplc) purity of >99%.

### Example 9. Preparation of

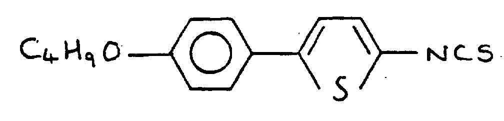

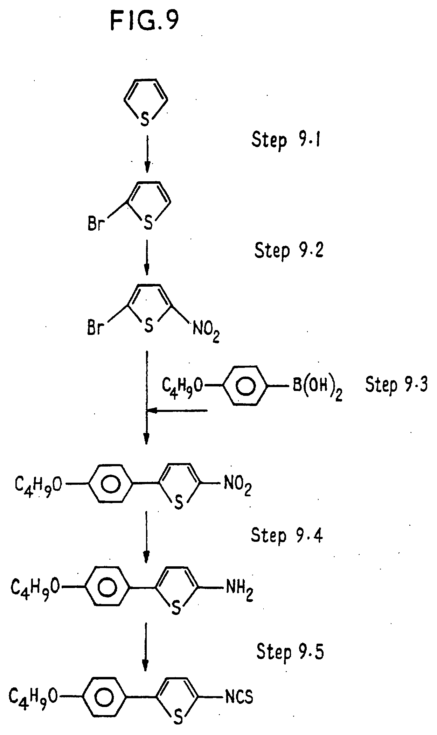

With reference to figure 9 it can be seen that

### Step 9.1 2-Bromothiophene.

A solution of thiophene (31.88g, 0.380mol) and N-bromosuccinimide (64.00g, 0.360mol) in a mixture of chloroform (80ml) and glacial acetic acid (80ml) was heated under reflux (with stirring) for 0.5 hours (constant glc analysis revealed a complete reaction with minimal formation of 2,5-dibromothiophene). The reaction mixture was diluted with water and washed with dichloromethane (2x100ml); the combined organic extracts were washed successively with water (300ml) and aqueous potassium hydroxide (5%, 300ml) before being dried (MgSO₄). The solvent was removed *in vacuo* and the residue was distilled to give a colourless liquid.
Yield 24.34g (42%).

### Step 9.2 2-Bromo-5-nitrothiophene.

Nitric acid (24.00g, 1.42 sp gr, 0.381mol) in acetic anhydride (50ml) at 0°C was added dropwise to a cooled (0°C) rapidly stirred solution of the compound of step 5.1 (24.77g, 0.152mol) in acetic anhydride (50ml). At the end of the addition the stirring was continued for 0.5 hr and the mixture was refrigerated overnight. The mixture was poured into ice water (400ml) and the precipitate was filtered off, dissolved in ether (2x200ml), and washed with water until free of acid. The solvent was removed *in vacuo* and the residue was purified by column chromatography [silica gel/petroleum fraction (bp 40-60°C), dichloromethane, 5:1] and was recrystallised from ethanol/dimethoxyethane, 100:1 to give a pale yellow solid which was dried *in vacuo* (P₂O₅) to give 66% yield of 20.89g.

### Step 9.3 2-(4-butoxyphenyl)5-nitrothiophene.

Quantities: 4-butoxy-1-phenylboronic acid (3.38g, 0.017mol), compound of compound of step 9.2 (3.31g, 0.016mol), tetrakis (triphenylphosphine) palladium(O) (0.910g, 0.001mol), sodium carbonate (15.8ml, 2.0M, 0.03mol).
The experimental procedure is carried out as described in step 8.3 above. The product was purified by column chromatography [silica gel/petroleum fraction (bp40-60°C), dichloromethane, 3:1] and was recrystallised from ethanol to give 4.57g (94% yield) of pale yellow solid which was dried *in vacuo* (P₂O₅).

### Step 9.4 2-Amino-5-(4-butoxyphenyl)thiophene.

Quantities: compound of step 9.3 (2.98g, 0.011mol), palladium on carbon (5%, 1.97g).

The experimental procedure is as described for the preparation of the compound of step 8.4 above, and gives a purple solid which can then be used in the next step without purification. Yield = 2.72g (100%).

### Step 9.5 2-(4-butoxyphenyl)-5-isothionatothiophene.

Quantities: compound of step 9.4 (2.72g, 0.011mol), thiophosgene (2.07g, 0.018mol), calcium carbonate (2.20g, 0.022mol).

The experimental technique and procedure is as described for 8.5 above. The product is then purified by column chromatography [silica gel/petroleum fraction (bp 40-60), dichloromethane, 5:1] and was recrystallised from hexane to give a white solid which was dried *in vacuo* (CaCO₃). Yield = 2.00g (63%).

Liquid crystal transition temperatures between crystalline (K), nematic (N), smectic B (S_{B}) and isotropic (I) are given in Table 1 below for compounds of Formula I and Formula II. The table also contains a comparison of anisotropy in polarisability Δ a with that of 4-cyano-(4'pentyl)-1-phenylcyclohexane (5PCH) and the birefringence (Δn) of the compounds. [ ] denotes a virtual phase transition.

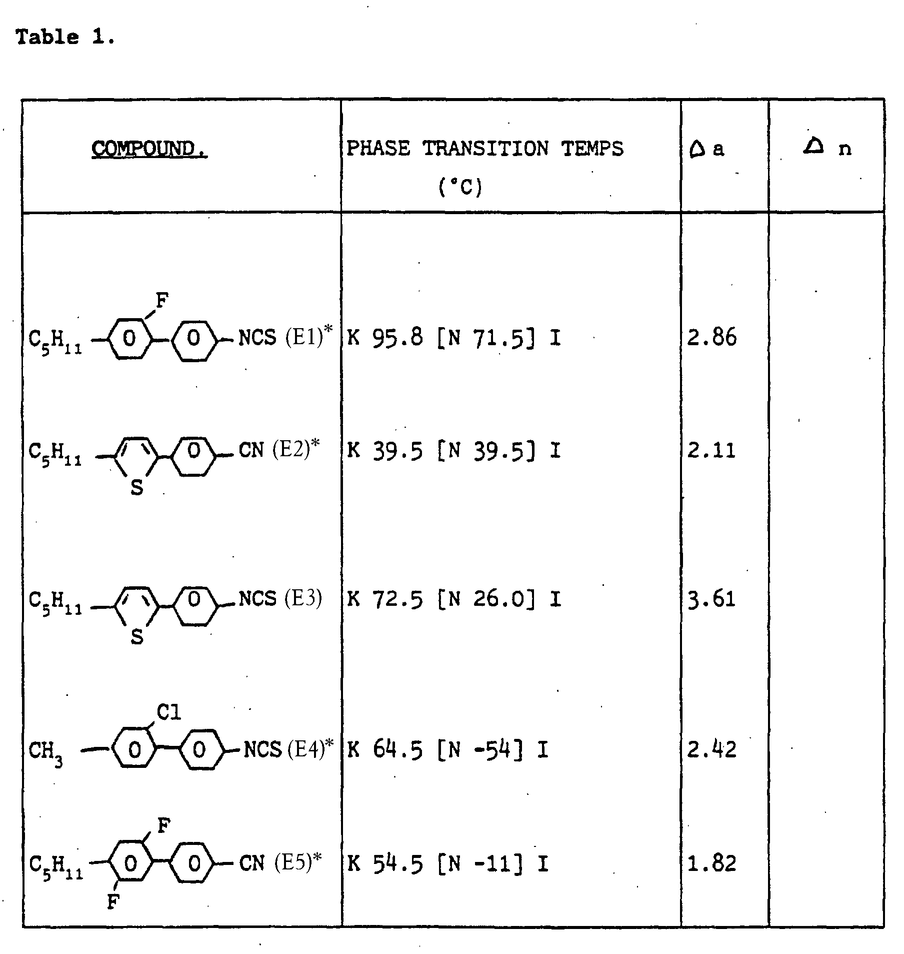

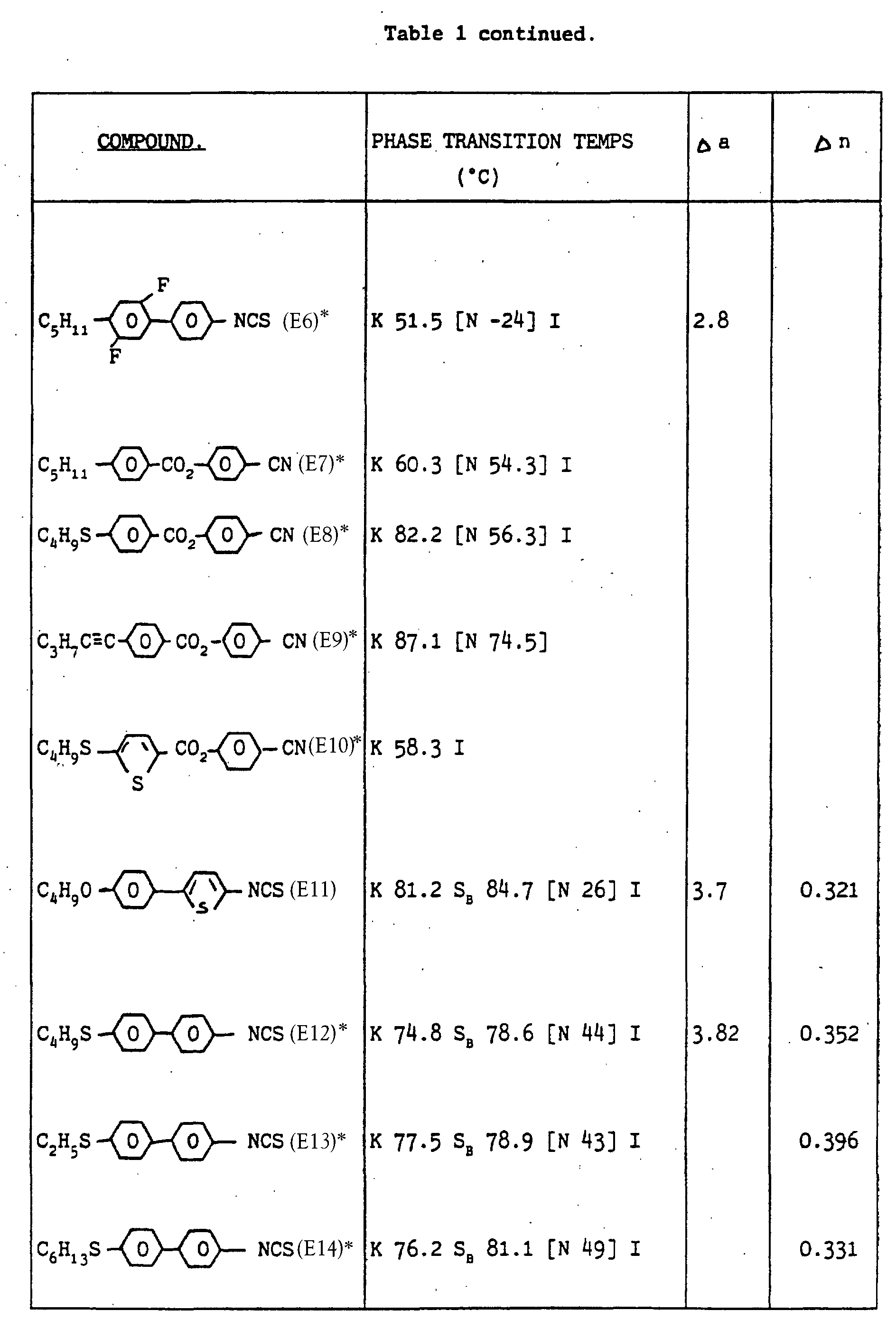

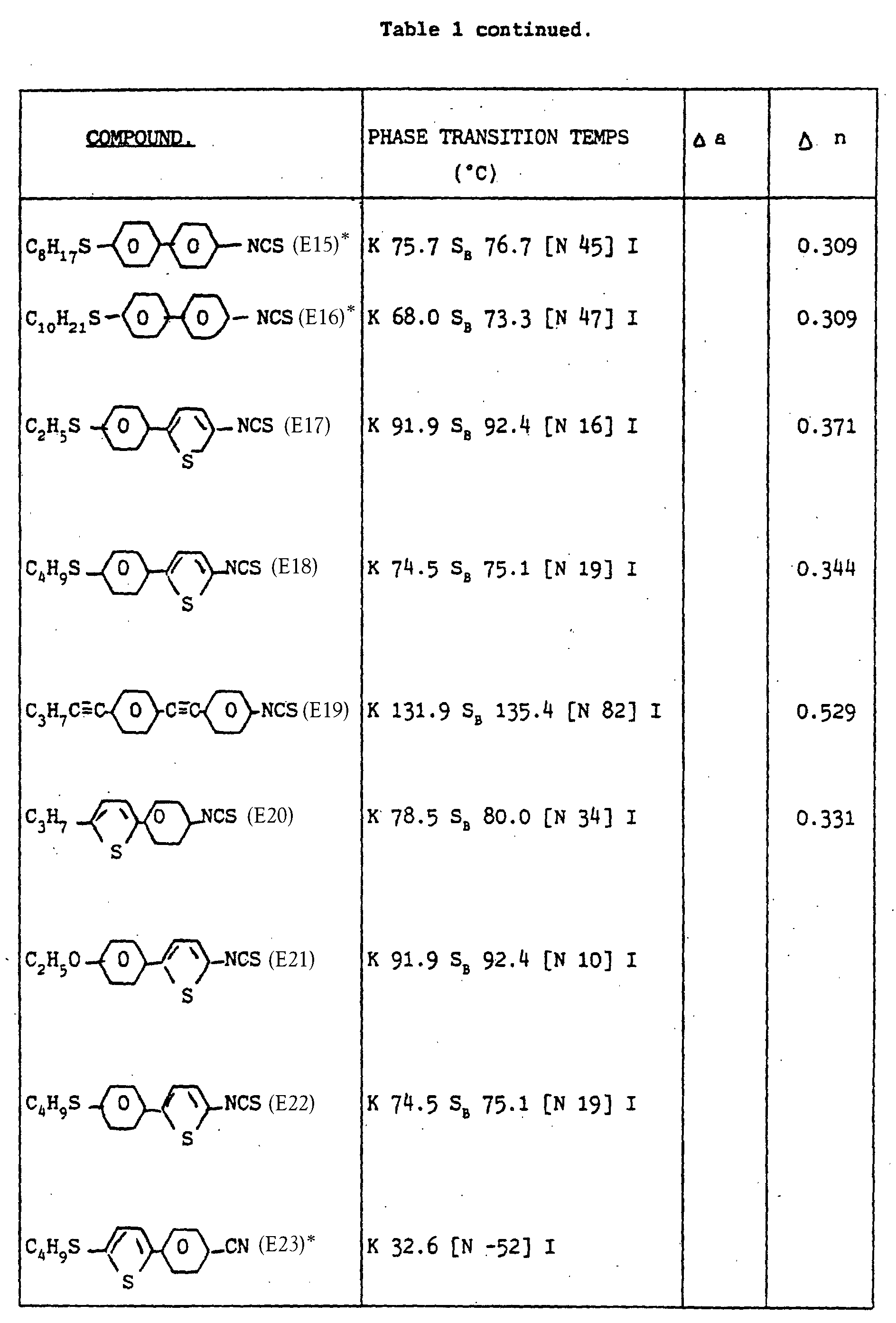

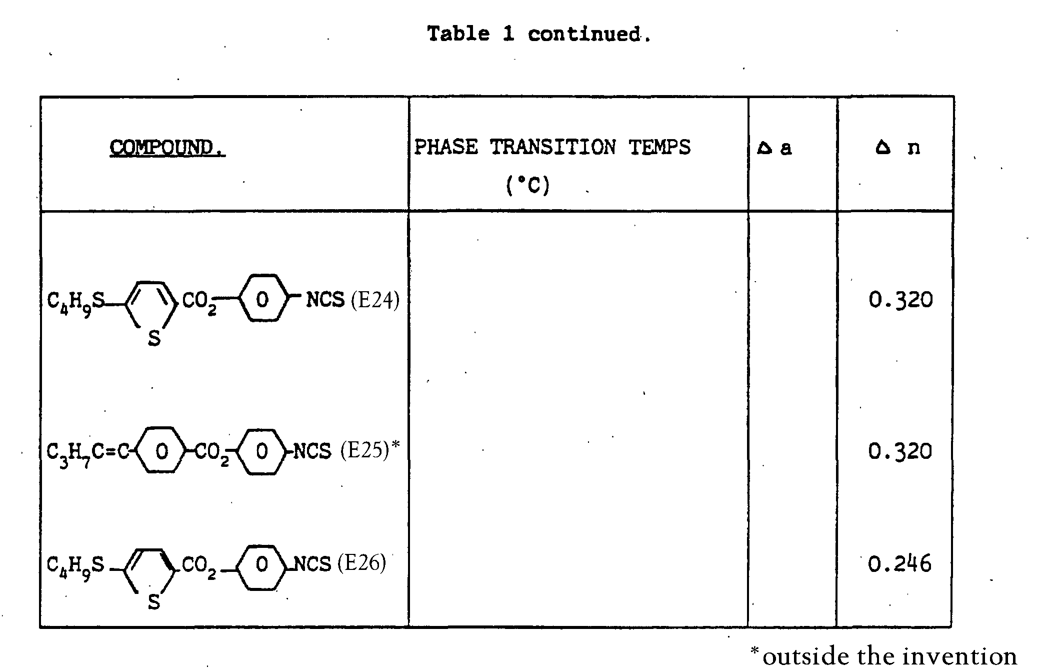

The Δn measurements are normalized for 25°C and were carried out using an Abbé refractometer and using 3 wt% of the compound to be measured in a non-polar eutectic nematic host, typically such as 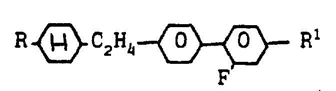 where R and R' are alkyl.

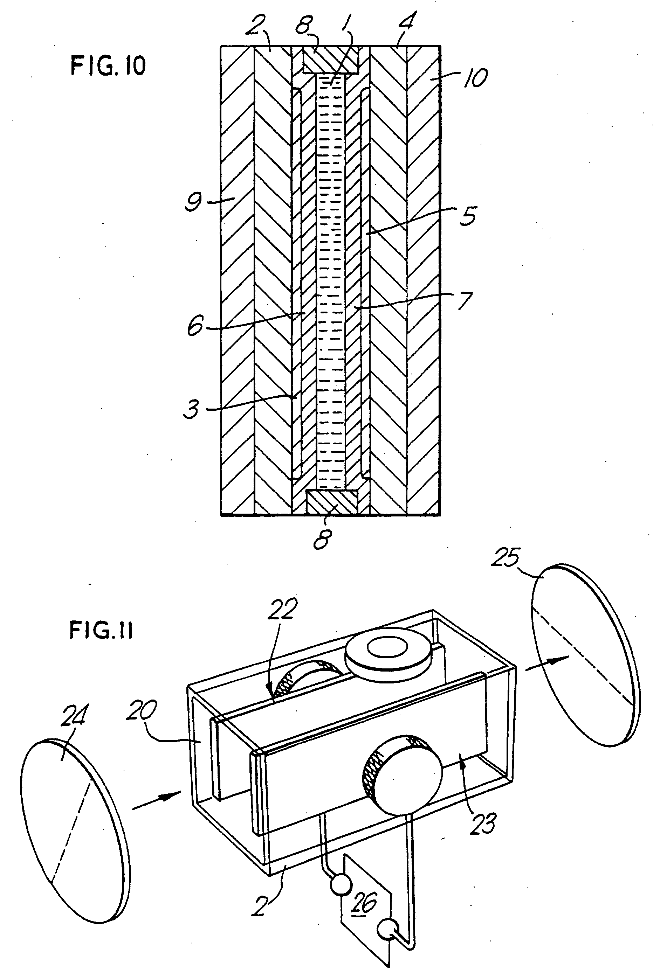

Figure 10 a liquid crystal cell comprises a layer 1 of liquid crystal material, where the material is a mixture incorporating compounds of formula I, sandwiched between a glass slide 2 having a conducting layer 3 on its surface, eg of indium tin oxide, and a glass slide 4 having a transparent conducting layer 5 on its surface. The slides 2,4 bearing the layers 3,5 are respectively coated with films 6,7 of a polyimide layer. Prior to construction of the cell the films 6 and 7 are rubbed with a soft tissue in a given direction, the rubbing directions being arranged parallel to the construction of the cell. A spacer 8 eg of polymethylmethacrylate, separates the slides 2,4 to the required distance eg 5 microns. The liquid crystal material 1 is introduced between the slides 2,4 by filling the space between the slides 2,4 and spacer 8 and sealing the spacer 8 in a vacuum in a known way.

A polarizer 9 is arranged with its polarization axis parallel to the rubbing direction on the films 6,7 and an analyzer (crossed polariser) 10 is arranged with its polarization axis perpendicular to that rubbing direction. When a voltage is applied across the cell by making contact with the layers 3 and 5 the cell is switched.

In an alternative device (not shown) based on a cell construction as shown in figure 10 the layers 3 and 5 may be selectively etched in a known way, eg by photoetching or deposition through a mask, eg to provide one or more display symbols, eg letters, numerals, words or graphics and the like as conventionally seen on displays. The electrode portions thereby may be addressed in a variety of ways which include multiplexed operation.

Figure 11 shows a Kerr cell 20. It comprises a glass cell 21 having two electrodes 22 and 23, which can be filled with a polar isotropic medium such as compounds of formula II or materials comprising mixtures including at least one compound of formula II. The cell 20 can be positioned between crossed linear polarisers 24 and 25, whose transmission axes are arranged to be at ± 45° to an applied electric field. Where there is zero voltage across the electrodes 22 and 23, and the cell 20 acts as a closed shutter. The application of a modulating electric field from voltage source 26 generates an electric field causing the cell 20 to act as a variable wave plate and thus operating as a variable aperture shutter where opening is proportional to the electric field.

## Claims

1. A liquid crystal compound **characterised by** Formula I: 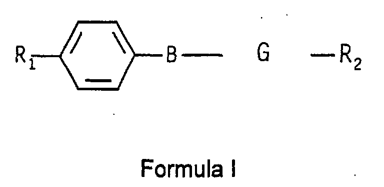 wherein R₁ is selected from alkyl, alkoxy, alkynyl, thioalkyl, hydrogen, NCS and SCN, R₂ is selected from alkyl, alkoxy, alkynyl, thioalkyl, hydrogen, CN, NCS and SCN; wherein alkyl, alkoxy, alkynyl, thioalkyl groups have from 1 to 15 carbon atoms; G is thiophene; B is selected from single bond, C≡C, C=C, COO, azoxy and diazo; provided that at least one of R₁ and R₂ is selected from SCN or NCS.

2. A liquid crystal compound **characterised by** Formula I wherein R₁ is CN, R₂ is selected from NCS and SCN; G is thiophene, B is selected from single bond, C≡C, C=C, azoxy and diazo.

3. A liquid crystal material, being a mixture of compounds, at least one of which is a compound as claimed in Claim 1 or Claim 2.

4. A liquid crystal material according to claim 3 and further **characterised by** the inclusion of at least one compound having a formula: 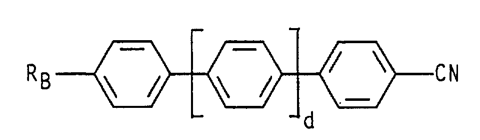 wherein R_{B} is alkyl, thioalkyl or alkoxy, preferably containing 1-8 carbon atoms, and preferably straight chain, and wherein d is 1 or 0.

5. A liquid crystal material according to claim 3 and further **characterised by** inclusion of at least one compound having a formula: 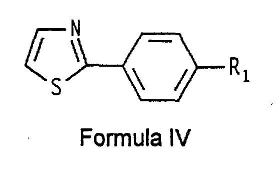 wherein R₁ is selected from CN, alkyl and alkoxy.

6. A liquid crystal device which uses a liquid crystal material according to claim 3.

7. A liquid crystal device utilising pretransitional characteristics comprising a liquid crystal compound according to either claim 1 or claim 2.

8. A liquid crystal device utilising pretransitional characteristics comprising a liquid crystal material according to one of claims 3 to 5.

## Patentansprüche

1. Flüssigkristalline Verbindung, die durch die Formel I 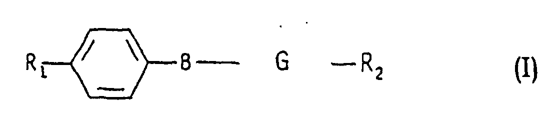 gekennzeichnet ist, in der bedeuten:
- R₁ einen Rest, der unter Alkyl, Alkoxy, Alkinyl, Thioalkyl, Wasserstoff, NCS und SCN ausgewählt ist,
- R₂ einen Rest, der unter Alkyl, Alkoxy, Alkinyl, Thioalkyl, Wasserstoff, CN, NCS und SCN ausgewählt ist,
worin die Alkyl-, Alkoxy-, Alkinyl-, Thioalkylreste 1 bis 15 Kohlenstoffatome aufweisen,
- G Thiophen,
- B eine Einfachbindung oder eine Gruppe, die unter C≡C, C=C, COO, Azoxy und Diazo ausgewählt ist,
mit der Maßgabe, daß mindestens einer der Reste R₁ und R₂ unter SCN und NCS ausgewählt ist.

2. Flüssigkristalline Verbindung der Formel I, in der R₁ CN, R₂ NCS oder SNC, G Thiophen und B eine Einfachbindung oder einen Rest, der unter C≡C, C=C, Azoxy und Diazo ausgewählt ist, bedeutet.

3. Flüssigkristallines Material, bei dem es sich um ein Gemisch von Verbindungen handelt, von denen mindestens eine Verbindung eine Verbindung nach Anspruch 1 oder Anspruch 2 ist.

4. Flussigkristallines Material nach Anspruch 3, **dadurch gekennzeichnet, daß** es mindestens eine Verbindung der Formel 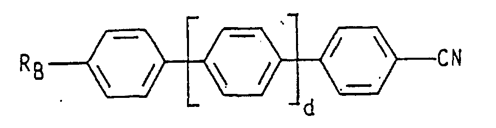 umfaßt, in der R_{B} Alkyl, Thioalkyl oder Alkoxy, das vorzugsweise 1 bis 8 Kohlenstoffatome aufweist und vorzugsweise geradkettig ist, bedeutet und in der d die Zahl 1 oder 0 darstellt.

5. Flüssigkristallines Material nach Anspruch 3, **dadurch gekennzeichnet, daß** es mindestens eine Verbindung der Formel IV 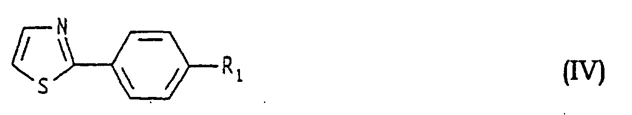 umfaßt, in der R₁ unter CN, Alkyl und Alkoxy ausgewählt ist.

6. Flüssigkristall-Vorrichtung, die ein flüssigkristallines Material nach Anspruch 3 enthält.

7. Flüssigkristall-Vorrichtung, die Vorphasenübergangseigenschaften ausnützt, die eine flüssigkristalline Verbindung nach Anspruch 1 oder Anspruch 2 enthält.

8. Flüssigkristall-Vorrichtung, die Vorphasenübergangseigenschaften ausnützt, die ein flüssigkristallines Material nach einem der Ansprüche 3 bis 5 enthält.

## Revendications

1. Composé à cristaux liquides, **caractérisé par** la formule I : 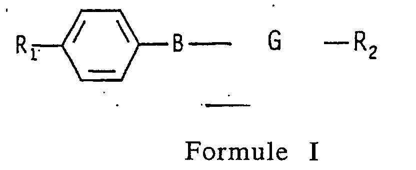 dans laquelle R₁ est choisi parmi les groupes alkyle, alcoxy, alcynyle, thioalkyle, hydrogène, NCS et SCN ; R₂ est choisi parmi les groupes alkyle, alcoxy, alcynyle, thioalkyle, hydrogène, CN, NCS et SCN ; les groupes alkyle, alcoxy, alcynyle, thioalkyle ayant de 1 à 15 atomes de carbone ; G est le thiophène ; B est choisi parmi une liaison simple, C≡C, C=C, COO, azoxy et diazo ; à la condition que l'un au moins de R₁ et R₂ soit choisi parmi SCN ou NCS.

2. Composé à cristaux liquides, **caractérisé par** la formule I dans laquelle R₁ est CN, R₂ est choisi parmi NCS et SCN, G est le thiophène et B est choisi parmi une liaison simple, C≡C, C=C, azoxy et diazo.

3. Matériau à cristaux liquides qui est un mélange de composés dont l'un au moins est un composé selon la revendication 1 ou la revendication 2.

4. Matériau à cristaux liquides selon la revendication 3, caractérisé en plus par l'inclusion d'au moins un composé ayant pour formule : 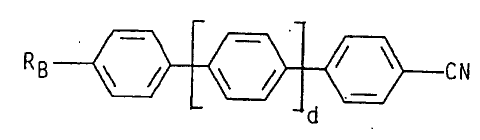 dans laquelle R_{B} est un groupe alkyle, thioalkyle ou alcoxy, de préférence contenant de 1 à 8 atomes de carbone et de préférence à chaîne droite, et dans laquelle d vaut 1 ou 0.

5. Matériau à cristaux liquides selon la revendication 3, caractérisé en plus par l'inclusion d'au moins un composé ayant pour formule : 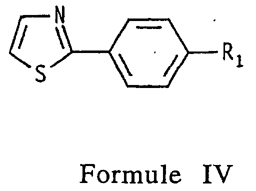 dans laquelle R₁ est CN, alkyle, ou alcoxy.

6. Dispositif à cristaux liquides utilisant un matériau à cristaux liquides selon la revendication 3.

7. Dispositif à cristaux liquides utilisant des caractéristiques prétransitionnelles, comprenant un composé à cristaux liquides selon la revendication 1 ou la revendication 2.

8. Dispositif à cristaux liquides utilisant des caractéristiques prétransitionnelles, comprenant un composé à cristaux liquides selon l'une des revendications 3 à 5.
